# FASCICULE DE BREVET EUROPEEN

(11) **EP 3 350 671 B1**
(45) Date de publication et mention de la délivrance du brevet: **17.11.2021**
(21) Numéro de dépôt: 16769979.2
(22) Date de dépôt: 19.09.2016
(51) Int. Cl.: G06F 3/01, A61B 3/11, A61B 3/113, A61B 3/032

(54) **PROCEDE DE DETERMINATION D'UN PARAMETRE OPHTALMOLOGIQUE**
VERFAHREN ZUR BESTIMMUNG EINES OPHTHALMOLOGISCHEN PARAMETERS
METHOD FOR DETERMINING AN OPHTHALMOLOGICAL PARAMETER

(30) Priorité: 18.09.2015 FR 1558825
(43) Date de publication de la demande: 25.07.2018
(73) Titulaire: Suricog, 75015 Paris (FR)
(72) Inventeur: MASSONNEAU, Marc, 27570 Tillieres Sur Avre (FR); SWYNGHEDAUW, Marc, 75019 Paris (FR); LE LIBOUX, Kristen, 75010 Paris (FR)
(74) Mandataire: Nony
(86) Numéro de dépôt international: PCT/EP2016/072161
(87) Numéro de publication internationale: WO 2017/046419

(56) Documents cités:
- CN-B- 102 961 119
- US-A1- 2006 077 558
- US-A1- 2015 042 558
- US-A1- 2015 131 051

## Description

La présente invention concerne la détermination de paramètres anatomiques utiles pour la conception et/ou le choix de montures et/ou de verres adaptés à un sujet.

Il est connu de mesurer les caractéristiques du visage d'un sujet par rapport à un modèle précis de montures choisi par le sujet, à l'aide d'une borne fixe ou d'une tablette. La société Essilor, avec ses technologies EyeCode et M'eye Fit Touch, les sociétés Acep, Zeiss, et Visionix, ou encore la société Hoya, avec sa technologie VisuReal+, proposent une telle approche.

La société Experoptic propose un kit de prise de mesures, comportant une monture à assembler destinée à être photographiée lorsqu'elle est portée par le sujet. Les mesures et photographies sont renseignées sur Internet, les lunettes fabriquées à distance, puis livrées.

La demande WO 2014/006516 A1 décrit un système comportant une caméra externe pour prendre une photographie afin de déterminer la distance pupillaire d'un sujet. La demande WO 2013/086137 A1 enseigne un procédé de détermination de la distance pupillaire d'un sujet par prise de photographie à l'aide d'une caméra placée devant le visage du sujet. Le brevet US 8 459 792 B2 divulgue un procédé de détermination de la distance pupillaire d'un sujet par prise d'une photographie après placement d'un support de référence sur son front.

Le brevet EP 2 062 090 B1 divulgue une caméra reliée à un ordinateur qui observe à distance un sujet muni d'une monture. La demande WO 2014/077462 A1 décrit un procédé de mesure d'angle de rotation de l'œil à l'aide d'une caméra externe.

Le document US 7 794 085 B2 décrit un dispositif permettant de déterminer le centre de rotation d'un œil d'un sujet à l'aide notamment d'une cible distante. Le brevet EP 2 400 880 B1 enseigne un procédé de détermination du centre de rotation de l'œil fondé sur la description de la surface de la cornée par un modèle mathématique, la surface de la cornée étant observée dans deux positions différentes par un dispositif distant.

La prise de mesures décrite dans ces documents est contraignante et nécessite la présence de dispositifs externes, non embarqués, qui observent à distance l'œil du sujet.

Du fait de leur caractère éloigné de l'œil du sujet, ces dispositifs externes entraînent également une certaine imprécision de la mesure.

La demande US 2006/0077558 divulgue un dispositif permettant de détecter la position de la pupille précisément. La distance interpupillaire peut être calculée.

US 2015/0131051 divulgue un autre dispositif pour calculer la position de la pupille pour l'identification de l'iris.

La demande US2015/0042558 divulgue un dispositif permettant de déterminer la direction du regard et configuré pour calculer en autres la position du centre de rotation de l'œil.

Le document CN 102 961 119 décrit un pupillomètre permettant de déterminer la position des pupilles en modifiant la transmittance des verres pour ne laisser passer la lumière que dans certaines zones correspondant aux pupilles.

Toutes ces demandes ne cherchent pas à délivrer des données relatives à des paramètres anatomiques dans le but de fabriquer des verres et/ou des montures, et en particulier ne cherchent pas à déterminer les demi-écarts pupillaires, qui sont le plus souvent différents à gauche et à droite chez un même individu et dont la connaissance est utile pour adapter au mieux les verres et/ou la monture à la personne.

Il existe donc un besoin pour simplifier la détermination de paramètres ophtalmologiques ainsi que pour améliorer la précision, dans le but notamment de sélectionner et/ou de fabriquer les verres d'une monture et/ou cette dernière..

### Procédé de détermination de paramètre(s) ophtalmologique(s)

L'invention est définie dans les revendications 1 - 9.

Le procédé de détermination selon l'invention permet un travail directement dans le contexte du visage, au plus proche de l'œil, améliorant ainsi la précision. Il ne nécessite pas d'observation distante. Il est compatible avec des systèmes informatiques standards tels que des ordinateurs, des tablettes, ou des smartphones.

### Dispositif placé sur le sujet

Le dispositif de suivi du regard peut être porté par le sujet par l'intermédiaire d'un dispositif placé sur le sujet comportant le dispositif de suivi du regard, le dispositif placé sur le sujet étant notamment une monture, un masque, un casque ou une sur-monture, en particulier une monture de type lunettes.

Le dispositif placé sur le sujet peut comporter un repère de positionnement destiné à être positionné de façon prédéfinie relativement au nez du sujet, notamment destiné à être aligné avec son axe médian.

### Dispositif de suivi du regard

Le dispositif de suivi du regard comporte au moins un capteur optique par œil. Ainsi le dispositif de suivi du regard peut comporter au moins un capteur optique gauche et au moins un capteur optique droit. Le capteur optique gauche observe l'œil gauche du sujet. Le capteur optique droit observe son œil droit.

Le dispositif de suivi du regard peut comporter deux capteurs optiques gauches et deux capteurs optiques droits. On peut ainsi reconstruire la profondeur, grâce à un système stéréoscopique.

La position des capteurs optiques gauche(s) et droit(s) sur le dispositif placé sur le sujet et/ou la distance entre ces capteurs est notamment connue. La position des capteurs optiques gauche(s) et droit(s) par rapport au repère de positionnement est notamment connue.

Les capteurs optiques peuvent être thermiques ou à rayonnement infrarouge. Les capteurs optiques peuvent être associés à des LEDs. Les capteurs optiques comportent une caméra, notamment RGB. Les caméras peuvent être thermiques.

Le dispositif de suivi du regard peut comporter au moins un éclairage droit, éclairant l'œil droit du sujet, et au moins un éclairage gauche, éclairant l'œil gauche du sujet, chaque éclairage étant notamment un éclairage visible et/ou infrarouge, de lumière polarisée ou non, en particulier filtrée en phase ou en amplitude, notamment structurée ou non, émettant notamment un motif lumineux prédéterminé, les capteurs optiques droit(s) et gauche(s) étant respectivement sensibles à l'éclairage droit et à l'éclairage gauche.

Le dispositif de suivi du regard comporte de préférence un système embarqué, sans fil.

Des dispositifs de suivi du regard adaptés à l'invention sont par exemple divulgués dans les demandes FR 2 989 482 et FR 3 011 952.

Le procédé de détermination d'un ou de plusieurs paramètres ophtalmologiques du sujet peut comporter une phase de calibration pour déterminer la position des capteurs optiques par rapport aux yeux du sujet.

On peut en particulier afficher sur un écran, transparent ou non, une ou plusieurs mires et/ou un ou plusieurs motifs destinés à être vus par le sujet, notamment pour guider son regard.

### Paramètres ophtalmologiques

Le procédé de détermination selon l'invention permet en particulier de déterminer des demi-écarts pupillaires du sujet et comporte l'observation des deux yeux du sujet par le dispositif de suivi du regard porté par le sujet. Le dispositif de suivi du regard peut permettre de détecter la position de la pupille sur chaque œil. La distance entre les capteurs optiques gauche et droit peut être connue ou calculée à partir de la configuration du dispositif placé sur le sujet. Le dispositif peut comporter un pont de nez ajustable en longueur, qui peut être utilisé pour la détermination des paramètres ophtalmologiques lorsqu'en place sur le sujet.

Les demi-écarts pupillaires gauche et droit sont mesurés par rapport à l'axe médian du nez du sujet. Ils peuvent être identiques ou non.

Les demi-écarts pupillaires peuvent être déterminés grâce notamment à la position des capteurs optiques par rapport au repère de positionnement. Les procédés de détermination de l'écart pupillaire du sujet, de ses demi-écarts pupillaires, et de la position de ses pupilles peuvent comporter une phase de calibration pour déterminer la position des capteurs optiques par rapport aux yeux.

Le procédé de détermination selon l'invention peut permettre également déterminer la position du centre de rotation des yeux du sujet.

La direction du regard du sujet peut se déduire de l'image des yeux du sujet grâce aux capteurs optiques. La pupille projetée sur un capteur optique donne une ellipse dont les caractéristiques sont reliées à cette direction 3D. Le centre de rotation de l'œil peut être déterminé en intersectant toutes les directions obtenues lors d'un parcours oculaire donné.

### Appareil de détermination de paramètre(s) anatomique(s) faciaux

### Moyens de détermination de la forme d'au moins une partie du nez du sujet

L'invention a également pour objet un appareil de détermination d'un ou de plusieurs paramètres anatomiques faciaux d'un sujet comportant une monture à placer sur le sujet, la monture comportant des moyens de détermination de la forme d'au moins une partie du nez du sujet, lesdits moyens de détermination délivrant une information représentative de ladite forme.

Les moyens de détermination de la forme d'au moins une partie du nez ont notamment une position connue sur la monture.

Au moins l'un des paramètres anatomiques faciaux, notamment l'ensemble des paramètres anatomiques faciaux, peut être choisi parmi la largeur et l'inclinaison locale du nez du sujet lorsque la monture est placée sur le sujet.

Les moyens de détermination de la forme d'au moins une partie du nez peuvent permettre de déterminer l'écartement du nez dans leur zone de contact avec le nez du sujet.

Les moyens de détermination de la forme d'au moins une partie du nez peuvent comporter deux ailettes pivotantes destinées à se placer chacune contre une aile du nez et un dispositif de réglage de l'écartement entre les ailettes.

Les moyens de détermination de la forme d'au moins une partie du nez peuvent comporter deux articulations reliant chacune le dispositif de réglage à une ailette. Les articulations peuvent comporter chacune une liaison pivot.

Les moyens de détermination de la forme d'au moins une partie du nez peuvent comporter deux capteurs mesurant chacun le déplacement en rotation d'une ailette par rapport au dispositif de réglage. Le déplacement en rotation des ailettes par rapport au dispositif de réglage de l'écartement entre les ailettes peut se faire de manière incrémentielle ou continue. Les capteurs mesurant le déplacement en rotation des ailettes par rapport au dispositif de réglage peuvent être des potentiomètres rotatifs qui transforment un angle en résistance électrique ou des codeurs optiques.

Le dispositif de réglage peut comporter une branche ajustable en longueur et un capteur mesurant le déplacement axial de la branche. Le déplacement axial de la branche ajustable en longueur peut se faire de manière incrémentielle ou continue. Le capteur mesurant le déplacement axial de la branche peut être un potentiomètre linéaire qui transforme une longueur en résistance électrique.

Les moyens de détermination de la forme d'au moins une partie du nez peuvent comporter une diode électroluminescente, une fibre optique épousant la forme d'au moins une partie du nez, et une photodiode qui transforme le rayonnement lumineux émis par la diode électroluminescente et transmis par la fibre optique en signal électrique, relié à la forme d'au moins une partie du nez.

En variante, les moyens de détermination de la forme d'au moins une partie du nez peuvent comporter un pont de nez choisi parmi un jeu de ponts de nez calibrés adaptés à des formes de nez différentes, chacun des ponts de nez, lorsqu'intégré à la monture, délivrant une information qui lui est propre.

L'information délivrée par le pont de nez, lorsqu'intégré à la monture, peut être une information binaire.

Le dispositif placé sur le sujet peut comporter un dispositif électronique permettant la reconnaissance automatique du pont de nez choisi.

Le pont de nez, lorsqu'intégré à la monture, peut constituer ou comporter un repère de positionnement tel que décrit précédemment.

Selon une autre variante, les moyens de détermination de ladite forme peuvent comporter au moins une bande souple déformable délivrant une information représentative de la déformation qu'elle subit.

La bande souple peut être un capteur de type « flex » transformant une déformation en résistance électrique.

La bande souple peut également être insérée dans un pont de nez déformable.

Au moins l'un du ou des paramètres anatomiques faciaux peut être l'angle des ailes du nez du sujet ou la largeur de son nez.

La monture peut comporter un dispositif de suivi du regard, comportant notamment un capteur optique. Le capteur optique peut comporter l'une ou plusieurs des caractéristiques décrites précédemment.

La monture peut comporter un dispositif de suivi du regard observant les deux yeux du sujet, permettant notamment la détermination d'un ou de plusieurs paramètres ophtalmologiques du sujet, choisi(s) parmi son écart pupillaire, ses demi-écarts pupillaires, la position du centre de rotation de ses yeux, et la position de ses pupilles lorsque son regard est à l'infini.

Le dispositif de suivi du regard peut comporter l'une ou plusieurs des caractéristiques décrites précédemment.

### Capteur sensible au contact avec une oreille

Selon un autre aspect, l'invention a pour objet un appareil de détermination d'un ou de plusieurs paramètres anatomiques faciaux d'un sujet, notamment tel que défini précédemment, comportant une monture à placer sur le sujet, la monture comportant deux branches latérales, au moins une branche comportant au moins un capteur sensible au contact avec une oreille du sujet lorsque la monture est placée sur le sujet, de préférence chacune des deux branches comportant un capteur sensible au contact avec une oreille du sujet lorsque la monture est placée sur le sujet, chaque capteur sensible au contact avec une oreille du sujet étant notamment un capteur de pression.

Le ou les capteurs sensibles au contact avec une oreille du sujet ont notamment une position connue sur la monture.

Le ou les capteurs sensibles au contact avec une oreille du sujet peuvent être sensibles également au contact avec le crâne du sujet.

Le ou les capteurs sensibles au contact avec une oreille du sujet peuvent être des capteurs de pression dont la résistance varie en fonction de la pression exercée.

### Capteur de divergence d'angle de branche latérale du sujet

L'invention a encore pour objet, selon un autre de ses aspects, un appareil de détermination d'un ou de plusieurs paramètres anatomiques faciaux d'un sujet, notamment tel que défini précédemment, comportant une monture, la monture comportant deux branches latérales et au moins un capteur de divergence d'angle de branche latérale, notamment un capteur de divergence d'angle de branche latérale pour chaque branche latérale.

Le(s) capteur(s) de divergence d'angle de branche latérale peuvent être des potentiomètres rotatifs qui transforment un angle en résistance électrique.

En variante, le(s) capteur(s) de divergence d'angle de branche latérale peuvent être des capteurs d'inclinaison, tels que par exemple le *Capteur d'inclinaison CMS (±0.25 V) 5 V typ. 4 mA Gamme(s) de mesure ±90 ° Murata SCA100T-D02* commercialisé par la société Conrad, ou des capteurs d'angle, tels que par exemple le *Set avec capteur d'angle*/*position et aimant AS500106 180* ° *alim 5 V*/*DC sortie 0.5* - *4.5 V Cherry Switches CU103602,* commercialisé par la société Conrad.

La monture peut comporter deux capteurs de divergence de branche latérale pour une branche latérale. La monture peut comporter deux capteurs de divergence de branche latérale pour chaque branche latérale.

La monture peut comporter une branche frontale et le ou les capteurs de divergence de branche latérale peuvent permettre de déterminer la position d'une ou des deux branches latérales par rapport à la branche frontale.

La monture peut comporter une première liaison pivot permettant le déplacement d'une branche latérale dans un premier plan sensiblement horizontal lorsque la monture est portée par le sujet. Le premier plan peut notamment comporter la branche frontale. Un premier capteur de branche latérale peut mesurer l'angle formé entre la branche frontale et la branche latérale dans le premier plan.

La monture peut comporter une seconde liaison pivot permettant le déplacement d'une branche latérale dans un second plan sensiblement vertical lorsque la monture est portée par le sujet. Le second plan peut notamment être perpendiculaire à la branche frontale. Un second capteur de branche latérale peut mesurer l'angle formé entre la branche frontale et la branche latérale dans le second plan.

La monture peut comporter une première liaison pivot et un premier capteur pour une même branche latérale, notamment pour chaque branche latérale. La monture peut comporter une seconde liaison pivot et un second capteur pour une même branche latérale, notamment pour chaque branche latérale.

La monture peut comporter des moyens de fixation pour fixer les branches latérales à la branche frontale.

Une procédure d'utilisation, comportant notamment des exercices à effectuer par le sujet, peut accompagner l'appareil, notamment en vue d'une calibration et/ou d'une prise en main optimale de l'appareil.

Au moins l'un du ou des paramètres anatomiques faciaux du sujet peut être la position relative de son nez par rapport à l'une de ses deux ou ses deux oreilles.

Au moins l'un des paramètres anatomiques faciaux peut être un paramètre ophtalmologique.

Une carte électronique, embarquant notamment des algorithmes de traitement, peut transmettre des informations liées aux paramètres anatomiques à un système tiers, par voie filaire ou sans fil.

### Procédé de mesure de paramètre(s) anatomiaue(s) faciaux

L'invention a également pour objet un procédé de mesure d'un ou de plusieurs paramètres anatomiques faciaux d'un sujet à l'aide d'un appareil de détermination d'un ou de plusieurs paramètres anatomiques faciaux d'un sujet selon l'invention.

L'appareil de détermination d'un ou de plusieurs paramètres anatomiques faciaux est notamment positionné sur le sujet de manière à s'adapter à celui-ci et à permettre aux paramètres anatomiques faciaux d'être mesurés.

Le procédé de mesure d'un ou de plusieurs paramètres anatomiques faciaux du sujet peut comporter une étape de calibration.

### Procédé de sélection et/ou de fabrication de verre(s) et/ou de monture

Selon un autre aspect, l'invention a pour objet un procédé de sélection et/ou de fabrication d'un ou de plusieurs verres et/ou d'une monture comportant la détermination d'un ou de plusieurs paramètres ophtalmologiques d'un sujet selon le procédé de détermination d'un ou de plusieurs paramètres ophtalmologiques d'un sujet selon l'invention et la sélection et/ou la fabrication d'un ou de plusieurs verres et/ou d'une monture au moins sur la base de cette détermination.

L'invention a également pour objet un procédé de sélection et/ou de fabrication d'un ou de plusieurs verres et/ou d'une monture, notamment tel que décrit précédemment, comportant la détermination d'un ou de plusieurs paramètres anatomiques faciaux tels que définis précédemment à l'aide d'un appareil selon l'invention, et la sélection et/ou la fabrication d'un ou de plusieurs verres et/ou d'une monture au moins sur la base de cette détermination.

Les verres peuvent être correcteurs et/ou solaires.

La détermination du ou des paramètres ophtalmologiques et/ou du ou des paramètres anatomiques faciaux peut ainsi être utilisée pour la fabrication de monture(s) et/ou de montage(s) adapté(e)(s) au sujet.

Par « montage » on entend l'opération d'adaptation des verres prescrits au sujet à la monture choisie par celui-ci. Il consiste essentiellement à retailler le verre avant sa fixation sur la monture, de sorte que le centre optique du verre coïncide avec la direction de regard à l'infini du sujet.

La détermination du ou des paramètres ophtalmologiques et/ou du ou des paramètres anatomiques faciaux peut permettre de personnaliser la monture, grâce par exemple à une adaptation des inclinaisons du plan de monture, des longueurs des branches, et/ou des écartements du pont de nez.

La détermination de la position des pupilles du sujet lorsque son regard est à l'infini dans le référentiel d'une monture telle que décrite précédemment, dite monture de référence, permet le montage sur une autre monture, dite monture finale, choisie par le sujet.

Selon une première variante, la monture de référence est conçue pour coïncider avec la monture finale, les facteurs de forme étant notamment identiques et les différences de design limitées aux finitions. Le montage peut être réalisé directement à partir des mesures prises sur la monture de référence. Il s'agit notamment de montures standardisées, en particulier lowcost.

Selon une seconde variante, la connaissance de la position de la monture de référence par rapport à la monture finale sur le visage du sujet est nécessaire pour passer du référentiel de la monture de référence à celui de la monture finale. Les techniques de modélisation 3D des montures et de scan 3D du visage peuvent permettre d'obtenir de telles informations.

Ainsi le procédé selon l'invention permet l'obtention de verres et de montures personnalisés. Il permet également de standardiser la fabrication de verres et de montures et donc d'engendrer un gain de temps et une réduction des coûts. La prescription et/ou la délivrance de montures et/ou de verres adaptés au sujet est facilitée.

### Figures

L'invention pourra être mieux comprise à la lecture de la description détaillée qui va suivre, d'exemples de mise en œuvre non limitatifs de celle-ci, et à l'examen du dessin sur lequel :
- la figure 1 représente un exemple d'appareil de détermination d'un ou de plusieurs paramètres anatomiques faciaux selon l'invention, et
- la figure 2 représente un autre exemple d'appareil de détermination d'un ou de plusieurs paramètres anatomiques faciaux selon l'invention.

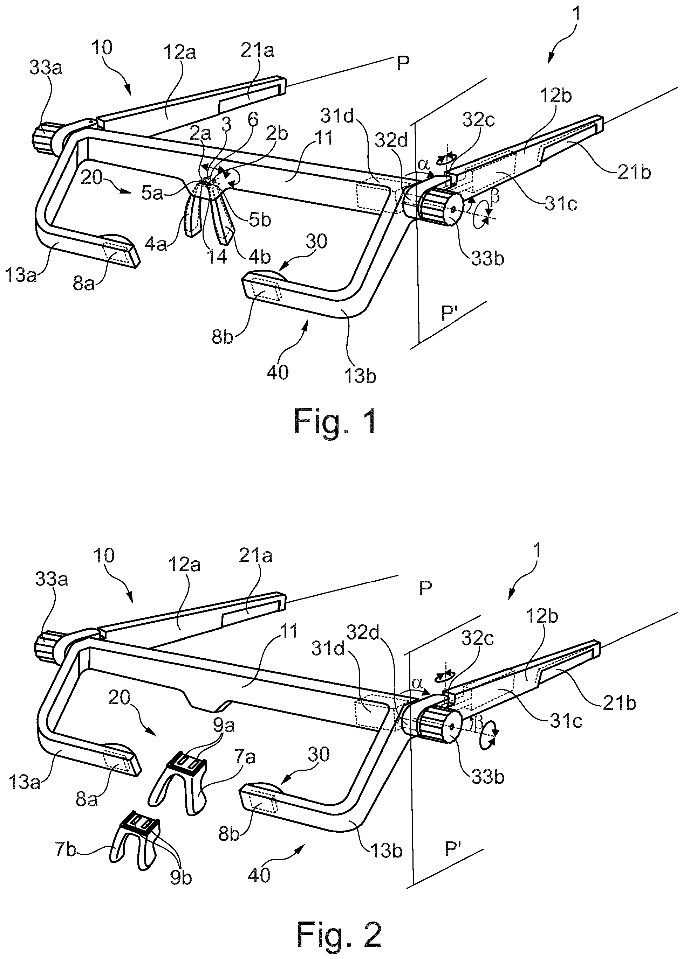

L'appareil de détermination 1 d'un ou de plusieurs paramètres anatomiques faciaux représenté sur la figure 1 comporte un dispositif 10 sous forme d'une monture à placer sur un sujet.

La monture 10 comporte une partie frontale 40 et deux branches latérales 12a et 12b. La partie frontale 40 comporte une branche frontale 11 et deux bras avants 13a et 13b.

La monture 10 comporte des moyens de détermination 20 de la forme d'au moins une partie du nez du sujet délivrant une information représentative de ladite forme.

Les moyens de détermination 20 de la forme d'au moins une partie du nez du sujet comportent deux ailettes pivotantes 4a et 4b destinées à se placer chacune contre une aile du nez du sujet, et un dispositif de réglage 3 de l'écartement entre les ailettes.

Les ailettes pivotantes 4a et 4b sont reliées au dispositif de réglage 3 par des liaisons pivot respectives 2a et 2b.

Les moyens de détermination 20 de la forme d'au moins une partie du nez du sujet peuvent comporter deux capteurs 5a et 5b mesurant respectivement le déplacement en rotation de l'ailette 4a et de l'ailette 4b par rapport au dispositif de réglage 3.

Les capteurs 5a et 5b sont par exemple des potentiomètres rotatifs qui transforment un angle en résistance électrique, tels que des capteurs de position résistifs trois broches 3382G-1-103G commercialisés par la société Bourns.

Les moyens de détermination 20 de la forme d'au moins une partie du nez du sujet peuvent comporter un capteur 6 mesurant le déplacement axial du dispositif de réglage 3.

Le capteur 6 mesurant le déplacement axial du dispositif de réglage 3 est par exemple un potentiomètre linéaire qui transforme une longueur en résistance électrique, tel que le potentiomètre slide 10k 45 mm PTA4543-2015DPB103 commercialisé par la société Bourns.

Le dispositif de réglage 3 de l'écartement entre les ailettes peut comporter un repère de positionnement 14 destiné à être aligné avec l'axe médian du nez du sujet.

Les moyens de détermination 20 de la forme d'au moins une partie du nez du sujet peuvent permettre de déterminer la largeur, l'inclinaison locale, et/ou l'angle des ailes du nez du sujet.

Les branches latérales 12a et 12b comportent des capteurs, respectivement référencés 21a et 21b, sensibles au contact avec une oreille du sujet lorsque la monture est placée sur le sujet.

Les capteurs 21a et 21b sensibles au contact avec une oreille du sujet sont par exemple des capteurs de pression, tels que les capteurs *Force Sensitive Resistor* ― *Small* ou *SoftPot Membrane Potentiometer* - *50mm* commercialisés par la société Sparkfun.

La monture 10 comporte également deux capteurs de divergence d'angle de branche latérale 31c et 31d. Ces capteurs 31c et 31d déterminent la position de la branche latérale 12b par rapport à la partie frontale 40 de la monture 10.

La monture 10 comporte une liaison pivot 32c permettant le déplacement de la branche latérale 12b dans un plan P comportant la branche frontale 11.

Le capteur de divergence d'angle de branche latérale 31c mesure l'angle α formé entre la branche frontale 11 et la branche latérale 12b dans le plan P comportant la branche frontale 11.

La monture 10 comporte une liaison pivot 32d permettant le déplacement de la branche latérale 12b dans un plan P' perpendiculaire à la branche frontale 11.

Le capteur de divergence d'angle de branche latérale 31d mesure l'angle β formé entre la branche frontale 11 et la branche latérale 12b dans le plan P' perpendiculaire à la branche frontale 11.

Les capteurs de divergence d'angle de branche latérale 31c et 31d sont par exemple des potentiomètres rotatifs qui transforment un angle en résistance électrique, tels que des capteurs de position résistifs trois broches 3382G-1-103G commercialisés par la société Bourns.

La monture 10 comporte des moyens de fixation 33a et 33b pour fixer les branches latérales respectives 12a et 12b à la partie frontale 40.

L'appareil 1 peut permettre de connaître la position relative du nez et des oreilles du sujet grâce à la connaissance de la zone de contact entre le nez du sujet et la monture 10 via les moyens de détermination 20 de la forme d'au moins une partie du nez du sujet, la connaissance de la zone de contact entre les oreilles du sujet et la monture via les capteurs 21a et 21b sensibles au contact avec une oreille du sujet et la connaissance de la configuration de la monture 10 via les capteurs de divergence d'angle de branche latérale 31c et 31d.

La monture 10 comporte également un dispositif de suivi du regard 30 comportant deux capteurs optiques 8a et 8b, à savoir un capteur optique droit 8a, orienté vers l'œil droit du sujet et un capteur optique gauche 8b, orienté vers son œil gauche.

Les capteurs optiques droit 8a et gauche 8b sont respectivement portés par les bras avant droit 13a et gauche 13b de la monture 10.

Le dispositif de suivi du regard 30, embarqué sur la monture 10, permet une observation des deux yeux du sujet.

L'appareil de détermination 1 d'un ou de plusieurs paramètres anatomiques faciaux permet de déterminer l'écart pupillaire du sujet, ses demi-écarts pupillaires, la position du centre de rotation de ses yeux, et/ou la position de ses pupilles lorsque son regard est à l'infini.

La figure 2 illustre une variante d'appareil de détermination 1 d'un ou de plusieurs paramètres anatomiques faciaux dans laquelle les moyens de détermination 20 de la forme d'au moins une partie du nez du sujet comportent un pont de nez 7a choisi parmi un jeu de ponts de nez calibrés 7a et 7b adaptés à des formes de nez différentes. Le sujet choisit le pont de nez 7a qui maximise son confort.

Les ponts de nez 7a et 7b comportent des moyens électroniques d'identification respectifs 9a et 9b.

Le pont de nez 7a, lorsqu'intégré à la monture 10, par exemple par encliquetage et/ou serrage, délivre une information qui lui est propre grâce à la reconnaissance de son moyen d'identification 9a par le reste de la monture 10.

Bien entendu, l'invention n'est pas limitée aux exemples illustrés et l'on peut notamment combiner entre elles des caractéristiques des exemples illustrés au sein de variantes non illustrées.

D'autres moyens de détermination 20 de la forme d'au moins une partie du nez du sujet sont possibles, par exemple des moyens comportant une bande souple déformable délivrant une information représentative de la déformation qu'elle subit, telle qu'un capteur de type flex, par exemple le Flex Sensor 2.2" commercialisé par la société Sparkfun. Le dispositif de suivi du regard 30 peut être porté par le sujet par l'intermédiaire d'un dispositif 10 placé sur le sujet autre qu'une monture, par exemple un masque, un casque ou une sur-monture. Un système stéréoscopique peut être utilisé pour reconstruire la profondeur, par exemple via un dispositif de suivi du regard 30 comportant deux capteurs optiques par œil. La monture 10 peut comporter un ou plusieurs capteurs de divergence d'angle de branche latérale pour chaque branche latérale.

## Revendications

1. Procédé de détermination d'au moins un paramètre ophtalmologique d'un sujet consistant en ses demi-écarts pupillaires, comportant l'observation des deux yeux du sujet par un dispositif de suivi du regard (30) porté par le sujet et la détermination dudit paramètre au moins à partir de ladite observation, le dispositif de suivi du regard (30) comportant au moins un capteur optique gauche (8b) et au moins un capteur optique droit (8a), les capteurs optiques comportant une caméra.

2. Procédé de détermination selon la revendication 1, le dispositif de suivi du regard (30) étant porté par le sujet par l'intermédiaire d'un dispositif (10) placé sur le sujet comportant ledit dispositif de suivi du regard (30), ledit dispositif (10) placé sur le sujet étant notamment une monture, un masque, un casque ou une sur-monture, en particulier une monture de type lunettes.

3. Procédé de détermination selon l'une quelconque des revendications précédentes, ledit dispositif (10) placé sur le sujet comportant un repère de positionnement (14) destiné à être positionné de façon prédéfinie relativement au nez du sujet, notamment destiné à être aligné avec son axe médian.

4. Procédé de détermination selon l'une quelconque des revendications précédentes, le dispositif de suivi du regard (30) comportant deux capteurs optiques gauches (8b) et deux capteurs optiques droits (8a).

5. Procédé de détermination selon l'une quelconque des revendications précédentes, le dispositif de suivi du regard (30) comportant au moins un éclairage droit et au moins un éclairage gauche, chaque éclairage étant notamment un éclairage visible et/ou infrarouge, de lumière polarisée ou non, en particulier filtrée en phase ou en amplitude, notamment structurée ou non, émettant notamment un motif lumineux prédéterminé, les capteurs optiques droit(s) (8a) et gauche(s) (8b) étant respectivement sensibles à l'éclairage droit et à l'éclairage gauche.

6. Procédé de détermination selon l'une quelconque des revendications précédentes, dans lequel on détermine la position du centre de rotation des yeux du sujet.

7. Procédé de détermination selon l'une quelconque des revendications précédentes, dans lequel on détermine la position des pupilles du sujet lorsque son regard est à l'infini.

8. Procédé de détermination selon l'une quelconque des revendications précédentes, dans lequel on affiche sur un écran, transparent ou non, une ou plusieurs mires et/ou un ou plusieurs motifs destinés à être vus par le sujet, notamment pour guider son regard.

9. Procédé de sélection et/ou de fabrication d'un ou de plusieurs verres et/ou d'une monture comportant la détermination d'au moins les demi-écarts pupillaires d'un sujet selon le procédé selon l'une quelconque des revendications 1 à 8 et la sélection et/ou la fabrication d'un ou de plusieurs verres et/ou d'une monture au moins sur la base de cette détermination.

## Patentansprüche

1. Verfahren zur Bestimmung mindestens eines ophtalmologischen Parameters eines Subjekts, der aus dessen halben Pupillendistanzen besteht, aufweisend das Betrachten der zwei Augen des Subjekts durch eine Blickfolgevorrichtung (30), die vom Subjekt getragen wird, und das Bestimmen des Parameters wenigstens anhand des Betrachtens, wobei die Blickfolgevorrichtung (30) mindestens einen linken optischen Sensor (8b) und mindestens einen rechten optischen Sensor (8a) aufweist, wobei die optischen Sensoren eine Kamera aufweisen.

2. Bestimmungsverfahren nach Anspruch 1, wobei die Blickfolgevorrichtung (30) vom Subjekt mittels einer Vorrichtung (10) getragen wird, die auf das Subjekt aufgesetzt ist, die die Blickfolgevorrichtung (30) aufweist, wobei die Vorrichtung (10), die auf das Subjekt aufgesetzt ist, insbesondere ein Gestell, eine Maske, ein Helm oder ein Obergestell, insbesondere ein Brillengestell ist.

3. Bestimmungsverfahren nach einem der vorhergehenden Ansprüche, wobei die Vorrichtung (10), die auf das Subjekt aufgesetzt ist, einen Positionierungsbezugspunkt (14) aufweist, der dazu bestimmt ist, auf vorgegebene Weise bezogen auf die Nase des Subjekts positioniert zu sein, insbesondere dazu bestimmt ist, an deren Mittelachse ausgerichtet zu sein.

4. Verfahren zur Bestimmung nach einem der vorhergehenden Ansprüche, wobei die Blickfolgevorrichtung (30) zwei linke optische Sensoren (8b) und zwei rechte optische Sensoren (8a) aufweist.

5. Bestimmungsverfahren nach einem der vorhergehenden Ansprüche, wobei die Blickfolgevorrichtung (30) mindestens eine rechte Beleuchtung und mindestens eine linke Beleuchtung aufweist, wobei jede Beleuchtung insbesondere eine sichtbare und/oder Infrarotbeleuchtung mit Licht, das polarisiert ist oder nicht, ist, das insbesondere phasen― oder amplitudengefiltert, insbesondere strukturiert ist oder nicht, die insbesondere ein vorgegebenes Lichtmotiv abgibt, wobei die rechte(n) (8a) und linke(n) (8b) optischen Sensoren jeweils für die rechte und linke Beleuchtung empfindlich sind.

6. Bestimmungsverfahren nach einem der vorhergehenden Ansprüche, wobei die Position des Drehpunkts der Augen des Subjekts bestimmt wird.

7. Bestimmungsverfahren nach einem der vorhergehenden Ansprüche, wobei die Position der Pupillen des Subjekts bestimmt wird, wenn dessen Blick auf unendlich ist.

8. Bestimmungsverfahren nach einem der vorhergehenden Ansprüche, wobei auf einem Bildschirm, der transparent ist oder nicht, ein oder mehrere Muster und/oder ein oder mehrere Motive angezeigt werden, die dazu bestimmt sind, vom Subjekt gesehen zu werden, insbesondere um dessen Blick zu führen.

9. Verfahren zur Auswahl und/oder Fertigung eines oder mehrerer Gläser und/oder eines Gestells, das das Bestimmen wenigstens der halben Pupillendistanzen eines Subjekts gemäß dem Verfahren nach einem der Ansprüche 1 bis 8 und das Auswählen und/oder Fertigen eines oder mehrerer Gläser und/oder eines Gestells wenigstens auf der Grundlage dieser Bestimmung aufweist.

## Claims

1. A method of determining at least one ophthalmological parameter of a subject consisting in their interpupillary half-distances, including observation of both eyes of the subject by a gaze tracking device (30) worn by the subject and determination of said parameter at least from said observation, the gaze tracking device (30) including at least one left optical sensor (8b) and at least one right optical sensor (8a), the optical sensors comprising a camera.

2. The determination method as claimed in claim 1, the gaze tracking device (30) being worn by the subject by means of a device (10) placed on the subject including said gaze tracking device (30), said device (10) placed on the subject being in particular a frame, a mask, a helmet or an overframe, in particular a spectacles type frame.

3. The determination method as claimed in any one of the preceding claims, said device (10) placed on the subject including a positioning marker (14) intended to be positioned in a predefined manner relative to the nose of the subject, in particular intended to be aligned with its median axis.

4. The determination method as claimed in any one of the preceding claims, the gaze tracking device (30) including two left optical sensors (8b) and two right optical sensors (8a).

5. The determination method as claimed in any one of the preceding claims, the gaze tracking device (30) including at least one right light and at least one left light, each light emitting in particular visible and/or infrared light, polarized or not, in particular filtered in phase or in amplitude, in particular structured or not, in particular emitting a predetermined light pattern, the right optical sensor(s) (8a) and left optical sensor(s) (8b) being respectively sensitive to the right light and to the left light.

6. The determination method as claimed in any one of the preceding claims, in which the position of the rotation center of the eyes of the subject is determined.

7. The determination method as claimed in any one of the preceding claims, in which the position of the pupils of the subject is determined when their gaze is focused at infinity.

8. The determination method as claimed in any one of the preceding claims, in which there are displayed on a transparent or non-transparent screen one or more test patterns and/or one or more patterns intended to be seen by the subject, in particular to guide their gaze.

9. A method of selecting and/or manufacturing one or more lenses and/or a frame including the determination of at least the interpupillary half-distances of a subject by the method as claimed in any one of claims 1 to 8 and the selection and/or the manufacture of one or more lenses and/or a frame at least on the basis of that determination.
